(19) Europäisches Patentamt
European Patent Office
Office européen des brevets

(11) Publication number : 0 590 998 A1

(12) EUROPEAN PATENT APPLICATION

(21) Application number : 93307813.1

(22) Date of filing : 01.10.93

(51) Int. Cl.[5] : C07C 49/217, C07C 49/255, A61K 7/48

(30) Priority : 01.10.92 JP 286872/92

(43) Date of publication of application : 06.04.94 Bulletin 94/14

(84) Designated Contracting States : DE FR GB IT

(71) Applicant : SHISEIDO COMPANY LIMITED
5-5 Ginza 7-chome
Chuo-ku Tokyo 104 (JP)

(72) Inventor : Okazaki, Tomomi, c/o Shiseido Research Centre
1050, Nippa-cho, Kohoku-ku
Yokohama-shi, Kanagawa 223 (JP)
Inventor : Kato, Mikiko, c/o Shiseido Research Centre
1050, Nippa-cho, Kohoku-ku
Yokohama-shi, Kanagawa 223 (JP)
Inventor : Matsushita, Yuji c/o Shiseido Research Centre
1050, Nippa-cho, Kohoku-ku
Yokohama-shi, Kanagawa 223 (JP)
Inventor : Uehara, Keeichi, c/o Shiseido Research Centre
1050, Nippa-cho, Kohoku-ku
Yokohama-shi, Kanagawa 223 (JP)

(74) Representative : Perry, Robert Edward
GILL JENNINGS & EVERY Broadgate House 7 Eldon Street
London EC2M 7LH (GB)

# (54) 1-Styryl-3-t-butyl-1,3-propanedione derivative and external preparation for skin using the same.

(57) Compounds having excellent UV-A absorptivity and a non-polar oil compatibility, and which can be used topically for treating the skin, are of the formula

$$(R)_nPh\text{-}CH{=}CH\text{-}CO\text{-}CH_2\text{-}CO\text{-}C(CH_3)_3$$

wherein $(R)_nPh$ represents phenyl optionally substituted by up to 3 groups R independently selected from $C_{1-8}$ alkyl and $C_{1-18}$ alkoxy.

EP 0 590 998 A1

[FIELD OF THE INVENTION]

This invention relates to a 1-styryl-3-t-butyl-1,3-propanedione derivative and an external preparation for skin and more particularly, a derivative having UV-A ultraviolet absorbency, and an external preparation for skin using the same.

[BACKGROUND ART]

Various influences of ultraviolet rays to skin, such as acceleration of skin aging, have recently been well known The ultraviolet rays included in sunlight are classified into a long wavelength ultraviolet ray having a wavelength from 400 nm to 320 nm as UV-A, a medium wavelength ultraviolet ray having a wavelength from 320 nm to 290 nm as UV-B, and a short wavelength ultraviolet ray having a wavelength of not more than 290 nm as UV-C in a field of skin science.

While most of the ultraviolet rays usually irradiated to a human body originate in sunlight, the UV-C are absorbed by the ozonosphere and the body is mainly influenced by the UV-A and UV-B.

Among these, the UV-B forms erythema or blister and causes aggravation of melanin formation and chromatosis when the skin is irradiated with the UV-B over a certain level.

On the other hand, the UV-A causes browning, decreasing of elasticity of skin, generation of wrinkles and acceleration of skin aging. Furthermore, UV-A promotes the erythema reaction and reinforce the reaction for some patients, and can be trigger of a light poison or a light allergy.

In order to protect the human skin from the UV-A trouble, various types of UV-A absorbent have been developed.

The prior UV-A absorbents used for practical application include a benzophenone derivative, a dibenzoylmethane derivative and a benzotriazol derivatives.

By the way, a nonpolar oily base such as liquid paraffin, squalane and silicone oil are recently used for external preparations for skin including the ultraviolet absorbents because the oily bases have not only water protectivity, but also excellent usability such as good spreadability, plain feeling and non stickiness.

The prior types of UV-A absorbents, however, are poor of solubility in the nonpolar oily base.

Furthermore, the UV-A absorbents are generally colored crystal, and have demerits such as crystallization in products under low temperature condition and straining clothes. Therefore, content of the UV-A absorbents is limited and it is not possible to present enough absorptivity of the UV-A light.

[DISCLOSURE OF THE INVENTION]

Accordingly, it is an object of the present invention to eliminate the above-described problems in the prior art and to provide a nonpolar base soluble material having a high UV-A ultraviolet ray absorptivity.

As a result of studies undertaken by the inventors so as to attain this aim, it has been found that a new 1-styryl-3-t-butyl-1,3-propanedione derivative had an excellent UV-A ultraviolet ray absorptivity as well as usability. The present invention has been achieved on the basis of this finding.

The 1-styryl-3-t-butyl 1,3-propanedione derivative according to this invention is represented by the following general structural formula (1),

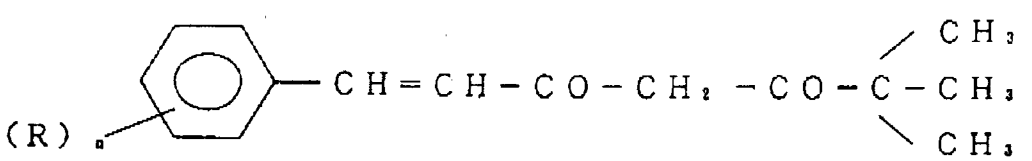 ···(1)

(In the general structural formula (1) above, R represents an alkyl group having 1 to 8 carbon atoms or alkoxyl group having 1 to 18 carbon atoms. n represents integer 0 to 3)

The external preparation for skin according to Claim 2 is characterized in that at least one of the 1-styryl-3-t-butyl 1,3-propanedione derivatives represented by the general structural formula (1) are contained therein.

Detail composition of this invention will be explained hereinafter.

In the general structural formula (1), R represents an alkyl group having 1 to 8 carbon atoms or an alkoxyl group having 1 to 18 carbon atoms. The alkyl or alkoxyl group may be any of a straight chain group or a branched chain group.

As examples of the alkyl group. the straight chain alkyl group such as a methyl group, an ethyl group. a propyl group, a butyl group. a pentyl group, a hexyl group, a heptyl group and an octyl group. and the branched chain alkyl group such as an isopropyl group. an isobutyl group, a t-butyl group, a neopentyl group, a 2-ethylbutyl group and a 2-ethylhexyl group can be cited.

As examples of the alkoxyl group, alkoxyl groups correspond to said alkyl groups. a branched alkoxyl group such as a 2-hexyldecyloxy group, a 2-heptylundecyloxy group and a methyl branched isostearyloxy group, and a straight chain alkoxyl group such as a dodecyloxy group, a tetradecyloxy group, a hexadecyloxy group and an octadecyloxy group. n represents number of substituents and 0 to 3 integer.

The 1-styryl-3-t-butyl 1,3-propanedione derivatives according to the present invention can be produced by the Claisen condensation between lower alkyl cinnamate and methyl-t-butylketone (Reaction formula 2). The Claisen condensation was described in "ORGANIC REACTIONS" No,8, p.59 John Wiley and Sons Inc.. New York 1954.

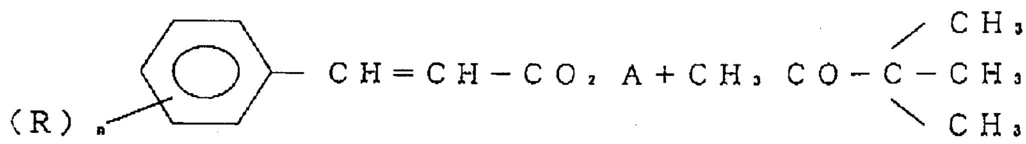 ···(2)

(In the formula, R represents the group defined in the formula 1. A represents lower alkyl group having 1 to 5 carbon atoms.)

As examples of solvents using for the reaction, benzene, toluene, xylene, hexane, diethylether, tetrahydrofuran, dioxane, dimethylformamide, and dimethylsulfoxide are cited.

Also, as examples of catalysts, sodium alcoholate of lower alcohol (1 to 4 carbon atoms), sodium amide. sodium hydroxide, metal sodium, sodium hydride and potassium hydroxide can be cited.

Reaction temperature is preferably 0 °C to boiling temperature of the solvents.

The derivatives thus obtained are liquid or solid having low melting point Maximal light absorbance of the derivatives are in UV-A area and can be use as UV-A absorbents.

Any bases which dissolve the 1-styryl-3-t-butyl 1,3-propanedione derivatives can be used as the bases of the external preparations for skin. Especially, nonpolar base such as liquid paraffin, squalane, silicone oil is preferably used because of excellent usability such as water proof ability, good spreadability, plain feeling and non stickiness, and is hardly removed by perspiration or water.

In case of using silicone base, as examples of the silicone base, chain polysiloxane such as dimethylpolysiloxane. methyl polysiloxane and methyl hydrogen polysiloxane, cyclo polysiloxane such as decamethyl polysiloxane, dodecamethyl polysiloxane, tetramethyl polysiloxane, denatured siloxane such as polyether denatured polysiloxane, fatty acid denatured polysiloxane, amino denatured polysiloxane and higher alcohol denatured polysiloxane can be cited.

The derivatives can appropriately be mixed with other components for ordinal cosmetics or pharmaceutical drugs. As examples of such components, oily components such hydrocarbon, fats and oils as liquid paraffin, squalane, vaseline. cetyl alcohol. isostearyl alcohol, cetyl 2-ethylhexanoate, 2-octyldodecyl alcohol, glycerin triisostearate, Macademian nuts oil, and lanolin; wax, silicone, surfactants, thickeners, neutralizers, antiseptics. germicides, anti-oxidants, powder components, pigments, perfumes, other ultraviolet absorbents, drugs, metallic sealant, and pH modifiers will be cited.

Also, types of the external preparations for skin, for example, a powder, a cream, a past. a stick, a liquid, a spray or a foundation may be utilized and an emulsified type using emulsifier may also be utilized.

The 1-styryl-3-t-butyl-1,3-propanedione derivatives can present enough UV-A absorptivity itself. However, it is possible to add other UV-B absorbents such p-dimethylaminobenzoate derivatives as 2-ethylhexyl p-dimethyl aminobenzoate, such p-methoxycinnamate derivatives as neoheparin (Harman and laymer corporation), salicylic acid derivatives, urocanic acid derivatives, such inorganic pigment as titanium dioxide and zinc oxide, and other UV-A absorbents.

The content of the derivatives according to the present invention, which can be changed according to the type of the preparation or necessary ultraviolet absobancy, is 0.1 to 20 weight % and more preferably 0.5 to 10 weight % in the preparation.

[EXAMPLES]

Embodiments of the present invention will be described hereinafter. It should be noted that the embodiments are not intended to limit the scope of the present invention. A unit for mixing rate is weight %.

Solubilities of the following compounds 1 to 5 as examples of the 1-styryl-3-t-butyl-1,3-propanedione derivative and 2-hydroxy-4-methoxybenzophenone as a comparison are examined using the SILICONE KF56 (10cs ; SHINETHU KACAKU INC.) as silicone base and squalane.

Compound 1 : 1-styryl-3-t-butyl 1,3-propanedione
Compound 2 : 1-p-methoxystyryl-3-t-butyl 1,3-propanedione
Compound 3 : 1-p-(2-ethylhexyloxy)styryl-3-t-butyl 1,3-propanedione
Compound 4 : 1-p-ethylstyryl-3-t-butyl1,3-propanedione
compound 5 : 1-m-methylstyryl-3-t-butyl1,3-propanedione

The solubilities of the 10% compounds in room temperature (25°C) are shown in the Table 1. The compounds which can completely be dissolved in the solvents are indicated as ○.

Table 1

| | | SOLUBILITY | |
|---|---|---|---|
| COMPOUND | STATE | SILICONE KF56(10%) | SQUALANE(10%) |
| 1 | LIGHT YELLOW SOLID | ○ | ○ |
| 2 | LIGHT YELLOW SOLID | ○ | ○ |
| 3 | LIGHT YELLOW LIQUID | ○ | ○ |
| 4 | LIGHT YELLOW SOLID | ○ | ○ |
| 5 | LIGHT YELLOW SOLID | ○ | ○ |
| COMPARISON 1 | LIGHT YELLOW CRYSTAL | DEPOSITION | DEPOSITION |

As is clear from the result, it is understood that the compounds according to the present invention have excellent solubility in nonpolar oil.

<u>ULTRAVIOLET RAY ABSORPTION</u>

Ultraviolet ray absorptivities of the compound 1,3,5 and comparison (2-hydroxy-4-methoxybenzophenone ; UV-A absorbent) were measured according to the following method.

(Measuring Method)

The compound 1,3,5 and the comparison compound were dissolved in ethanol (99.5%) so as to the concentration being 10ppm, and absorption were measured by using quartz cell (10mm×10mm) and the spectrophotometer UVIDEC-610 (JASCO CORPORATION).

The result was shown in TABLE 2

Table 2

| WAVELENGTH | COMPOUND 1 | COMPOUND 3 | COMPOUND 5 | COMPARISON |
|---|---|---|---|---|
| 300 | 0.60 | 0.20 | 0.50 | 0.39 |
| 310 | 0.82 | 0.28 | 0.72 | 0.35 |
| 320 | 1.08 | 0.40 | 1.00 | 0.40 |
| 330 | 1.33 | 0.56 | 1.26 | 0.42 |
| 340 | 1.40 | 0.76 | 1.36 | 0.34 |
| 350 | 1.24 | 0.92 | 1.24 | 0.20 |
| 360 | 0.96 | 0.97 | 0.98 | 0.11 |
| 370 | 0.50 | 0.89 | 0.54 | 0.03 |
| 380 | 0.26 | 0.65 | 0.32 | 0.00 |

As is clear from the result, the compounds according to the present invention had excellent ultraviolet ray absorptivity comparing with the comparison, and the result indicated high sun proof effect of the compound according to the invention.

Example 1 Preparation of the 1-styryl-3-t-butyl 1,3-propanedione

1.08 g of sodium methoxide was dissolved in 50 ml of dried toluene. 10 ml of dried toluene solution of 2.0g methyl-tert-butylketon was added to the system under stirring and 80 °C condition. The stirring was continued for 1 hour at the same temperature, and 10 ml of dried toluene solution of 3.2 g of methyl cinnamate was dropped. After stirring for 4 hours at 80°C, the toluene was removed under reduced pressure. 100 ml of ice water was added to the residue, adjusted the pH to about 4 by adding 6N-hydrochloric acid, and the compound was extracted with diethylether and dried with sodium sulfate anhydride. After removing the solvent, the compound was purified by silicagel column chromatography (5% ethyl acetate - hexane mixture). and 1.3 g of light yellow solid was obtained.

m.p. : 98 ~ 99°C
$\lambda_{max}$ : 340 nm ($\varepsilon$ =32200)
MASSPECTRUM $M^+$m/e 230

Example 2 Preparation of the 1-p-methoxystyryl-3-t-butyl 1,3-propanedione

8.7 g of sodium hydride was dissolved in 100 ml of dried dimethylsulfoxide under stirring condition and maintained at 25°C. 30 ml of dried dimethylsulfoxide solution in which 19.2 g of methyl p-methoxy cinnamate and 10.5 g of methyl-t-butylketone were dissolved was dropped into the system under stirring and 25 °C condition. After the dropping, the stirring was continued for 2 hours at 25 °C. The system was added into 600 ml of ice water and adjusted the pH to about 4 by adding 6N-hydrochloric acid. The compound was extracted by diethyl ether. After removing the solvent, the compound was purified by silicagel column chromatography (eluted by 5% ethyl acetate-hexane mixture), and 5.7 g of light yellow solid was obtained.

m.p. : 105.5 ~ 106.5 °C
$\lambda_{max}$ ; 355 nm ($\varepsilon$ =39000)
MASSPECTRUM $M^+$m/e 260

Example 3 Preparation of the 1-p-(2-ethylhexyloxystyryl)-3-t-butyl1,3-propanedione

By using 100 ml of dried dimethylsulfoxide, 29.0 g of methyl p-(2-ethylhexyloxy) cinnamate, 10.5 g of methyl-t-butylketone and 5.2 g of sodium hydride, the same reaction as the example 2 was conducted. The compound was purified by silicagel column chromatography (eluted by 5% ethyl acetate-hexane mixture), and 5.8 g of light yellow liquid was obtained.

$\lambda_{max}$ : 355 nm ($\varepsilon$=34700)
MASSPECTRUM $M^+$m/e 358

Example 4 Preparation of the 1-p-ethylstyryl-3-t-butyl 1,3-propanedione

4.0 g of methyl p-ethylcinnamate was dissolved in 100 ml of tetrahydrofuran, 1.3 g of sodium hydride was added to the system. 10 ml of tetrahydrofuran soluticn of 2.0 g of methyl-t-butylketone was dropped into the system under reflux condition. After the reaction was continued for 7 hours under reflux condition, the solvent was removed under reduced pressure. The residue was added into 300 ml of ice water. After adjusting the pH of the system to about 4 by adding 6N-hydrochloric acid, ether extraction was conducted. After the extract was washed and dried, the compound was purified by silicagel column chromatography (eluted by 3% ethyl acetate-hexane mixture), and 1.9 g of light yellow solid was obtained.

m.p. : 64 ~ 65 °C
$\lambda_{max}$ : 346 nm ($\varepsilon$ =37150)
MASSPECTRUM $M^{+}$m/e 258

Example 5 Preparation of the 1-m-methylstyryl-3-t-buty 1,3-propanedione

By using 3.7 g of methyl m-methyl cinnamate, 2.0 g of methyl-t-butylketone and 1.3 g of sodium hydride, the same reaction as the example 4 was conducted. The compound was purified by silicagel column chromatography (eluted by 3% ethyl acetate-hexane mixture), and 1.8 g of light yellow solid was obtained.

m.p. : 46 ~ 47 °C
$\lambda_{max}$ : 343 nm ($\varepsilon$ =33180)
MASSPECTRUM $M^{+}$m/e 244

Examples of external preparations for skin including the 1-styryl-3-t-butyl-1,3-propanedione derivatives will be explained hereinafter.

EXAMPLE 6 SUNSCREEN COSMETIC (OILY TYPE)

(1) Decamethylcyclopentasiloxane 47.0%
(2) Dimethylpolysiloxane (10cp/25°C) 20.0
(3) Methyl phenyl polysiloxane (20cp/25°C) 18.0
(4) Silicone resin 10.0
(5) 2-ethylhexyl p-dimethylaminobenzoate 3.0
(6) 1-styryl-3-t-butyl 1.3-propanedione 2.0

<Manufacturing method>

The cosmetic was prepared by mixing the ingredients (1)-(6), dissolving and filtrating.

<Sunscreen effect>

Sunscreen effects of the EXAMPLE 6, and comparative example which includes 2-ethylhexyl p-dimethylamino benzoate (content : 5.0%) instead of the 1-styryl-3-t-butyl 1, 3-propanedione (ingredient (6)) were examined.

Namely, the two preparations were applied to the half side of bodies of 10 panels respectively for the use test in the beach. And then, questionaire investigation of the suntan condition and investigation of the skin trouble were conducted.

The result is shown in table 3.

TABLE 3

| | | EXAMPLE 6 | COMPARISON |
|---|---|---|---|
| Panel | A | ○ | ○ |
| | B | ○ | × |
| | C | △ | × |
| | D | ○ | △ |
| | E | △ | △ |
| | F | ○ | △ |
| | G | ○ | ○ |
| | H | ○ | △ |
| | I | △ | △ |
| | J | ○ | × |

| | | |
|---|---|---|
| Number of skin trouble | none | smarts : 2<br>itches : 3<br>eruption : 1 |

Evaluation standard
- Strong suntan was admitted : ×
- Suntan was admitted : △
- Suntan was hardly admitted : ○

As is clear from the result, the ultraviolet ray shield effect of the external preparation for skin including 1-styryl-3-t-butyl 1, 3-propanedione derivative was higher than that of the external preparation for skin including the conventional ultraviolet absorbent (2-ethyl hexyl p-dimethylamino benzoate) and safety is also high because of none skin trouble.

EXAMPLE 7 SUNSCREEN COSMETIC (W/O CREAM)

| | |
|---|---|
| (1) Octamethylcyclotetrasiloxane | 10.5% |
| (2) Dimethylpolysiloxane (100cs) | 5.0 |
| (3) Dimethylpolysiloxane (2,500,000cs) | 3.0 |
| (4) Liquid paraffin | 15.0 |
| (5) Polyether denatured silicon | 6.0 |
| (6) 2-ethylhexyl p-methoxycinnamate | 5.0 |
| (7) 1-p-methoxystyryl-3-t-butyl 1,3-propanedione | 4.0 |
| (8) Purified water | 43.1 |
| (9) Monosodium L-glutamate | 3.0 |
| (10) 1, 3-butylene glycol | 5.0 |
| (11) Preservatives | 0.2 |
| (12) Perfume | 0.2 |

<Manufacturing method>

An oil part was prepared by mixing, heating and dissolving the ingredients (1)-(7) and (12) and maintained the temperature thereof to 70 °C. An aqueous part was obtained by dissolving the ingredients (8)-(11) and heating at 70 °C. The aqueous part was added to the oil part and emulsified with an emulsifier. The system was stirred and cooled, and when the temperature became equal to or less than 35 °C, filled into a container.

EXAMPLE 8 SUNSCREEN COSMETIC (O/W CREAM)

| | |
|---|---|
| (1) Decamethylcyclopentasiloxane | 3.0 |
| (3) Liquid paraffin | 8.0 |
| (3) Isopropyl myristate | 2.0 |
| (4) Petrolatum | 4.0 |
| (5) Cetanol | 4.0 |
| (6) Stearic acid | 3.0 |
| (7) Glycerylmonoisostearate | 3.0 |
| (8) 2-ethylhexyl p-dimethylamino benzoate | 3.0 |
| (9) 1-m-isobutoxystyryl-3-t-butyl 1,3-propanedione | 1.0 |
| (10) Preservatives | 0.2 |
| (11) Perfume | 0.2 |
| (12) Glycerol | 10.0 |
| (13) Propylene glycol | 5.0 |
| (14) Hyaluronic acid | 0.01 |
| (15) Potassium hydroxide | 0.2 |
| (16) Purified water | 53.39 |

<Manufacturing method>

An oil part was prepared by mixing the ingredients (1)-(11) and heating at 70°C. An aqueous part was prepared by dissolving the ingredients (12)-(16) and heating at 70°C. The oil part was added to the aqueous part and emulsified by an emulsifier. The emulsion was cooled down to 30°C by a heat exchanger, and filled in container.

EXAMPLE 9 SUNSCREEN LOTION

| | |
|---|---|
| (1) Dimethylsiloxane (5cs) | 10.0% |
| (2) Methyl phenyl polysiloxane (20cs) | 7.0 |
| (3) Stearic acid | 1.0 |
| (4) 2-ethyl hexyl p-methoxy cinnamate | 5.0 |
| (5) 1-p-(2-ethylhexyloxy)styryl-3-t-butyl 1, 3-propanedione | 10.0 |
| (6) Preservatives | 0.2 |
| (7) Perfume | 0.2 |
| (8) Glycerol | 5.0 |
| (9) Montmorillonite | 0.5 |
| (10) Potassium hydroxide | 0.2 |
| (11) Purified water | 60.9 |

<Manufacturing method>

An oil part was prepared by stirring the ingredients (1)-(7) and heating at 70 °C. An aqueous part was prepared by stirring the ingredients (8)-(11) and heating at 70 °C. The oil part was added to the aqueous part and emulsified by an emulsifier. The emulsion was cooled down to 30 °C with a heat exchanger and the sunscreen lotion was obtained.

EXAMPLE 10 DUAL PURPOSE FOUNDATION

| | |
|---|---|
| (1) Silicone treated titanium oxide | 9.5% |
| (2) Silicone treated mica | 40.0 |
| (3) Silicone treated talc | 20.45 |
| (4) Silicone treated iron oxide | 7.5 |
| (5) Spherical nylon powder | 10.0 |
| (6) Trimethylolpropanetriisostearate | 5.0 |
| (7) Squalane | 3.0 |
| (8) Bees was | 2.0 |
| (9) 1-m-methylstyryl-3-t-butyl 1.3-propanedione | 0.5 |
| (10) Sorbitan trioleate | 1.0 |
| (11) Preservatives | 0.5 |
| (12) Vitamin E | 0.05 |
| (13) Perfume | 0.5 |

<Manufacturing method>

The ingredients (1)-(5) were mixed by the henchel mixer and the ingredients (6)-(7) were added therein. The product was pulverized and formed in a inner plate.

EXAMPLE 11 SUNSCREEN STICK COSMETIC

| | |
|---|---|
| (1) Titanium oxide | 10.0 |
| (2) Zinc oxide | 7.0 |
| (3) Mica | 16.0 |
| (4) Red iron oxide | 1.5 |
| (5) Yellow iron oxide | 1.5 |
| (6) Black iron oxide | 1.0 |
| (7) Dimethylsiloxane (20cs) | 29.4 |
| (8) Trimethylolpropane-tri-2-ethylhexanoate | 8.0 |
| (9) Liquid paraffin | 7.0 |
| (10) Microcrystalline wax | 2.0 |
| (11) Ceresin | 1.0 |
| (12) Solid paraffin | 6.0 |
| (13) 2-ethyl hexyl p-dimethylamino benzoate | 5.0 |
| (14) 1-p-ethylstyryl-3-t-butyl 1, 3-propanedione | 3.0 |
| (15) Perfume | 0.5 |
| (16) Antioxidant | 0.1 |
| (17) Sorbitansesquioleate | 1.0 |

<Manufacturing method>

The ingredients (1)-(6) were mixed by the henchel mixer. The ingredients (7)-(9), (13), (14), (16), (17) were stirred, heated and dissolved, and the mixture of the ingredients (1)-(6) was added therein. A dissolved mixture of the ingredients (10)-(12) and (15) was added into the above mixture and formed stick shape.

EXAMPLE 12 SUNSCREEN COSMETIC BASE

| | |
|---|---|
| (1) Squalane | 19.0 |
| (2) Glyceryltriisostearate | 10.0 |
| (3) ISOPER G | 5.0 |
| (4) Sorbitan sesquioleate | 1.0 |
| (5) Polysiloxane ethylene denatured organopolysiloxane | 3.0 |
| (6) Purified water | 45.0 |
| (7) 1,3-butylene glycol | 5.0 |
| (8) Fine particle titanium oxide | 10.0 |
| (9) 2-ethyl hexyl p-methoxy cinnamate | 1.0 |
| (10) 1-p-n-octylstyryl-3-t-butyl 1, 3-propanedione | 1.0 |
| (11) Preservatives | q.s. |
| (12) Antioxidant | q.s. |
| (13) Perfume | q.s. |

(Manufacturing method>

The ingredients (1)-(5), (9), (10), (12), (13) were stirred and dissolved at 70 °C, and a mixture of the ingredients (6)-(8) and (11), which were dissolved and heated at 70 °C, was added. The system was emulsified and cooled, and the base cosmetic was obtained.

As described above. the external preparation for skin according to the present invention can absorb ultraviolet rays in the UV-A area, and have excellent water proof. Therefore. bases and other ingredients can be freely chosen. Furthermore, the sunscreen cosmetics have excellent stability when the cosmetics are left under harsh condition such as under blazing sun.

Also, the external preparation for skin according to the present invention has merits such as good spreadability, plain feeling, non stickiness, and hardly removed with perspiration or water, and then it is possible to maintain the UV-A absorption for long time.

## Claims

1. A compound of the formula

$$(R)_nPh\text{-}CH{=}CH\text{-}CO\text{-}CH_2\text{-}CO\text{-}C(CH_3)_3$$

   wherein $(R)_nPh$ represents phenyl optionally substituted by up to 3 groups R independently selected from $C_{1-8}$ alkyl and $C_{1-18}$ alkoxy.

2. A compound according to claim 1, which is 6,6-dimethyl-1-phenyl-1-heptene-3,5-dione.

3. A compound according to claim 1, which is 6,6-dimethyl-1-(4-methoxyphenyl)-1-heptene-3,5-dione.

4. A compound according to claim 1, which is 6,6-dimethyl-1-[4-(2-ethylhexyloxy)phenyl)-1-heptene-3,5-dione.

5. A compound according to claim 1, which is 6,6- dimethyl-1-(4-ethylphenyl)-1-heptene-3.5-dione.

6. A compound according to claim 1, which is 6,6-dimethyl-1-(3-methylphenyl)-1-heptene-3,5-dione.

7. A topical composition which comprises one or more compounds according to any of claims 1 to 6, and a carrier acceptable to the skin.

8. A composition according to claim 7, which comprises a silicone base.

9. A cosmetic method for the treatment of skin, which comprises the topical administration of a composition according to claim 7 or claim 8.

10. Use of one or more compounds according to any of claims 1 to 6, for the manufacture of a composition for use in therapeutic treatment of the skin.

European Patent Office

# EUROPEAN SEARCH REPORT

Application Number
EP 93 30 7813

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, vol. 115, no. 15, 14 October 1991, Columbus, Ohio, US; abstract no. 158145, SASNOVSKIKH V Y ET AL 'Synthesis of.beta.-aminovinyl ketones by condensation of nitriles with methyl ketones' * abstract * & ZH. ORG. KHIM. (ZORKAE,05147492);90;VOL.26 (10);PP.2086-91 URAL. GOS. UNIV.;SVERDLOVSK;USSR (SU) --- | 1,2 | C07C49/217<br>C07C49/255<br>A61K7/48 |
| A | EP-A-0 239 105 (G.D. SEARLE & CO.) * page 17; claims * ----- | 1 | |

| TECHNICAL FIELDS SEARCHED (Int.Cl.5) |
|---|
| C07C |

The present search report has been drawn up for all claims

| Place of search | Date of completion of the search | Examiner |
|---|---|---|
| THE HAGUE | 30 November 1993 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)